(19) Europäisches Patentamt
European Patent Office
Office européen des brevets

(11) Veröffentlichungsnummer: **0 010 607**
**B1**

(12) # EUROPÄISCHE PATENTSCHRIFT

(45) Veröffentlichungstag der Patentschrift: **05.08.81**

(21) Anmeldenummer: **79103522.3**

(22) Anmeldetag: **19.09.79**

(51) Int. Cl.³: **C 07 D 249/18**, C 07 D 249/22, D 06 L 3/12, C 08 K 5/27 // C07D413/12

(54) **Triazolylstyryl-Verbindungen sowie deren Verwendung zum Weisstönen organischer Materialien.**

(30) Priorität: **30.09.78 DE 2842686**

(43) Veröffentlichungstag der Anmeldung: **14.05.80 Patentblatt 80/10**

(45) Bekanntmachung des Hinweises auf die Patenterteilung: **05.08.81 Patentblatt 81/31**

(84) Benannte Vertragsstaaten: **CH DE FR GB**

(56) Entgegenhaltungen:
**EP-A-0 004 897**
**CH-A-523 900**
**CH-A-523 901**
**DE-A-1 805 371**
**DE-A-1 955 068**

(73) Patentinhaber: **BAYER AG, Zentralbereich Patente, Marken und Lizenzen, D-5090 Leverkusen 1, Bayerwerk (DE)**

(72) Erfinder: **Dorlars, Alfons, Dr., Mozartstrasse 32, D-5090 Leverkusen (DE)**
Erfinder: **Schellhammer, Carl-Wolfgang, Dr., Katharinental 26, D-5060 Bergisch-Gladbach 2 (DE)**

EP 0 010 607 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Triazolylstyryl-Verbindungen sowie deren Verwendung zum Weisstönen organischer Materialien

Gegenstand der vorliegenden Erfindung sind Verbindungen der Formel

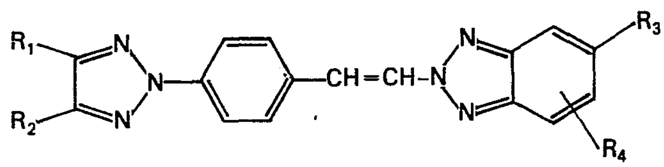

(I)

in welcher $R_1$ für Wasserstoff, $C_1$–$C_4$-Alkyl oder $R_2$,
$R_2$ für Phenyl, Tolyl, Chlorphenyl, Cyclohexyl, Xylyl, Biphenylyl oder Styryl stehen, und
$R_3$ und $R_4$ gemeinsam einen angular anellierten Benzolring bilden.

Bei den genannten Alkylgruppen handelt es sich insbesondere um $CH_3$ und $C_2H_5$.

Besonders bevorzugte Verbindungen sind solche der Formel (I), in der $R_1$ für Wasserstoff, $C_1$–$C_2$-Alkyl oder Phenyl,
$R_2$ für Phenyl oder Styryl stehen, und
$R_3$ und $R_4$ gemeinsam für die restlichen Glieder eines ankondensierten Benzolrings stehen.

Die neuen Triazolylstyrylverbindungen können auf verschiedene Weise hergestellt werden, indem man beispielsweise Benzotriazolylstyryl-oximinohydrazone der Formel

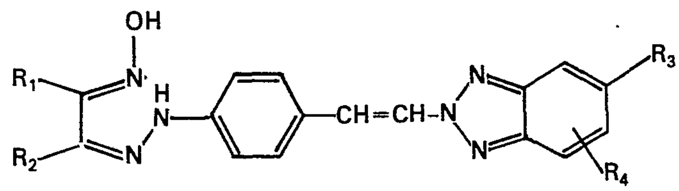

(II)

entweder direkt zu I dehydratisiert oder mit Dehydrierungsmitteln in die I-analogen Triazol-N-oxide der Formel

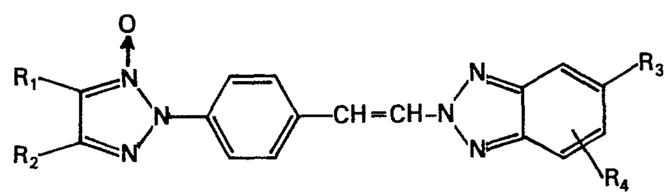

(III)

umwandelt und diese darauf zu den Triazolen I reduziert.

Beide Reaktionen sind an sich bekannt (vgl. DE-OS 1 917 740 = GB-PS 1 289 365 und DE-OS 2 258 276 = GP-PS 1 422 621).

Beispielsweise wird die Cyclodehydratisierung von II durch Einwirkung von Säureanhydriden, wie Acetanhydrid oder sonstigen Cyclisierungsmitteln wie Harnstoff oder Isocyanaten in geeigneten Lösungsmitteln, z.B. überschüssigem Acetanhydrid, Dimethylformamid, Dimethylsulfoxid, Glykolmethylether-acetat und anderen polaren aprotonischen Lösungsmittel herbeigeführt. Erforderlich ist dabei die Anwesenheit von Basen wie Natrium- oder Kaliumacetat, Triethylamin, Dimethylbenzylamin, Pyridin u.a.

Die Cyclodehydrierung der Oximinohydrazone II zu den betreffenden Triazol-N-oxiden III wird in solchen Lösungsmitteln ausgeführt, die gegenüber den jeweils gewählten Oxidationsmitteln und Reakionsbedingungen inert sind, z.B. Pyridin, techn. Pyridinhomologe, Dimethylformamid, Dimethylsulfoxid, Essigsäure und deren Gemische mit Wasser. Als Oxidationsmittel kommen hier in Frage: Kupfer-II-Salze, wie Kupferacetat, Kupferformiat, Kupfersulfat, komplexe Kupfer-II-Verbindungen, ferner Bleioxid, Bleitetraacetat, Kaliumhexacyanato-ferrat, sowie Wasserstoffperoxid oder Luftsauerstoff in Kombination mit Kupfer-II-Verbindungen. Die Reduktion der Triazol-N-oxide II zu den Triazolen I lässt sich leicht durch Einwirkung von Zinkstaub in essigsaurer oder salzsaurer Lösung bzw. Suspension bewerkstelligen. Auch durch Katalyse aktivierter Wasserstoff ist unter milden Reaktionsbedingungen geeignet.

Die für die erwähnten Umsetzungen erforderlichen Ausgangsverbindungen II lassen sich dadurch erhalten, dass man 2-[4-Hydrazinostyrol]-arylotriazole der Formel

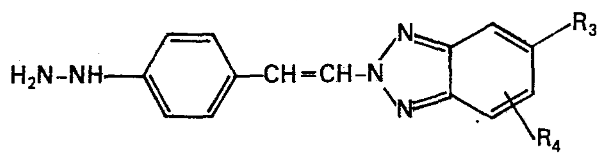

(IV)

mit α-Oximinoketonen der Formel

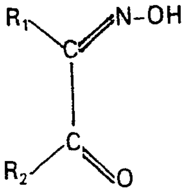

(V)

kondensiert. Diese Kondensation erfolgt zweckmässig in organischen Lösungsmitteln, insbesondere Alkoholen oder wasserhaltigen Alkoholen unter Säurekatalyse (Essigsäure, Phosphorsäure u.a.) zwischen Raumtemperatur und ca. 90 °C. Geeignete Oximinoketone sind z.B. in der oben zitierten Patentliteratur beschrieben.

Verbindungen der Formel (I), in der $R_1$ einen Alkylrest und $R_2$ einen Alkyl- oder Arylrest bedeuten, lassen sich zweckmässigerweise dadurch herstellen, dass man diazotierte 2-[4-Aminostyryl]-arylotriazole der Formel

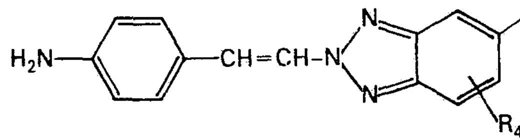

(VI)

mit Alkyl-isoxazolinonen der Formel

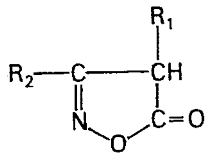

(VII)

kuppelt und die erhaltenen Azoverbindungen der Formel

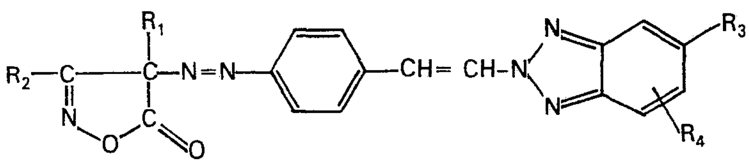

(VIII)

unter Decarboxylierung in die Triazole der Formel (I) umwandelt. Auch diese Reaktion ist im Prinzip bekannt.

Die Kupplung der gegebenenfalls im Benzolring des Benzotriazols substituierten 2-p-Aminostyrylbenzotriazole auf die durch $R_1$ und $R_2$ substituierten Isoxazolin-5-one (VII) erfolgt zweckmässig in essigsaurem Milieu. Die dabei als Zwischenprodukte erhaltenen Azoverbindungen (VIII) werden in einem organischen Lösungsmittel wie Methylenchlorid, Chloroform oder Xylol gelöst bzw. suspendiert und zu einer 60–130 °C warmen Mischung aus einem tertiären Amin und einem sekundären Alkohol, z.B. zu einer Mischung aus Isopropanol und Triethylamin oder aus Butanol-2 und Dimethylbenzylamin getropft; statt eines derartigen Gemisches kann man auch 1-Dimethylamino-propanol-2 oder Tris-isopropanolamin verwenden. Die gewünschten Reaktionsprodukte I scheiden sich meist beim Konzentrieren der Reaktionsmischung kristallin ab.

Ein vorteilhaftes Verfahren zur Herstellung von I ist ferner die Kondensation von v-Triazolylbenzaldehyden der Formel

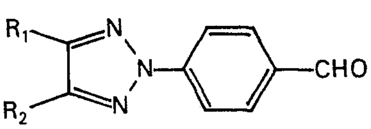

(IX)

mit Arylotriazolyl-(2)-essigsäuren der Formel

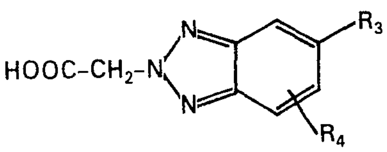

(X)

Diese an sich ebenfalls bekannte Kondensation (vgl. DE-OS 1955066 = US-PS 3728339) wird zweckmässig in Gegenwart basischer Katalysatoren unter den Bedingungen der Knoevenagel-Reaktion ausgeführt.

Die Triazolylbenzaldehyde sind z.T. aus der DOS 1917740 und der DOS 2258276 bekannt. Alle anderen Triazolylbenzaldehyde können in analoger Weise hergestellt werden, z.B. durch Rosenmund-Reduktion der betreffenden Triazolylbenzoylchloride oder durch Sommelet-Reaktion der betreffenden Triazolylbenzylchloride.

Die Arylotriazolyl-essigsäuren X sind ebenfalls grösstenteils bekannt bzw. in an sich bekannter Weise erhältlich (vgl. z.B. Tetrahedron 26, 497 f (1970) japanische Patentpublikationen 47/34621 und 42/76310).

Die neuen v-Triazolylstyryl-arylotriazolverbindungen I sind gelbstichig bzw. grünstichig-weisse bis blassgrüne kristalline Substanzen, die eine gute Affinität zu hochmolekularen synthetischen organischen Materialien aufweisen und sich in organischen Lösungsmitteln farblos mit starker blauer bis rotstichig blauer Fluoreszenz lösen. Sie eignen sich daher vorzüglich zum Aufhellen, bzw. Weisstönen von Fäden, Geweben, Folien und anderen Gebilden aus Polyolefinen, Polyacrylnitril, Polyvinylchlorid, Polyamiden und besonders von Polyestern, wie z.B. Polyterephthalsäureglykolester.

Sie lassen sich in üblicher Weise anwenden, z.B. in Form wässriger Dispersionen oder Lösungen und Dispersionen in organischen Lösungsmitteln, wie z.B. Ethylenglykolmonomethylether, Dimethylformamid, Diglykolmethylether, Triethanolamin u.a.

Ferner können sie in Schmelz- und Spinnmassen eingearbeitet werden, die zur Herstellung

von Fasern, Folien und Formkörpern dienen. Die anzuwendenden Mengen lassen sich durch Vorversuche leicht ermitteln; sie liegen im Bereich von 0,01 bis 0,5%, bezogen auf das Gewicht des aufzuhellenden Materials.

Gegenüber nächstvergleichbaren Verbindungen gemäss EP-A 0004897 zeichnen sich die erfindungsgemässen Triazolylstyrylverbindungen durch den Vorteil der besseren Aufhellwirkung auf Polyesterfasern aus.

Beispiel 1

132 g 4-[4-Phenyl-5-methyl-v-triazolyl-(2)]-benzaldehyd werden zusammen mit einer äquimolaren Menge an Naphthotriazolyl-(2)essigsäure in 300 ml trockenem techn. Xylol verrührt. Nach Zugabe von 8,5 g Piperidin und 6,0 g Essigsäure wird das Gemisch unter Rühren zunächst auf 100°, dann innerhalb von einer Stunde auf Siedetemperatur erhitzt, wobei man über einen Wasserabscheider unter Rückfluss des Xylols das Reaktionswasser abtrennt. Nach Beendigung der Wasserabspaltung (ca. 2 Stunden) destilliert man das Xylol unter langsamem Überleiten von Stickstoff ab, wobei man die Temperatur der Schmelze bis 170° steigen lässt. Man gibt weitere 8,5 g Piperidin hinzu und rührt bis zur Beendigung der Kohlendioxid-Entwicklung, die man durch Einleiten des Abgases in Calciumhydroxidlösung feststellt. Nach Abkühlen auf etwa 160° lässt man 80 ml Essigsäure und 170 ml Methylglykol einfliessen, wobei das gebildete Triazolylstyrol auskristallisiert. Man saugt das Produkt bei ca. 10° ab, wäscht es mit wenig kaltem Methylglykol und Methanol, reinigt es durch Umkristallisieren aus Methylglykol und trocknet. Man erhält weisse Kristalle der Formel

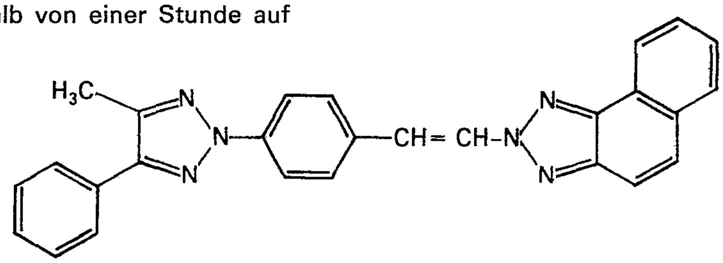

In analoger Weise wurden aus den entsprechenden v-Triazolyl-benzaldehyden und den entsprechenden Arylotriazolylessigsäuren die in der folgenden Tabelle aufgeführten Verbindungen I erhalten, welche in Dimethylformamidlösung bei Bestrahlung mit UV-Licht von 350 nm die in der letzten Spalte angegebene Fluoreszenzfarbe haben.

Auf Polyesterfasern erhält man mit diesen Verbindungen Aufhellungen von guter Licht-, Nass- und Chloritechtheit.

| Formel-Nr. | $R_1$ | $R_2$ | A | Farbe |
|---|---|---|---|---|
| 1 | 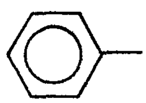 | H | 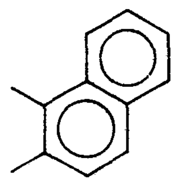 | rotstichig blau |
| 2 | 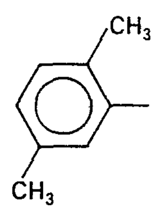 | $CH_3$ | 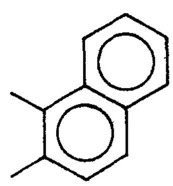 | etwas rotstichig blau |
| 3 | 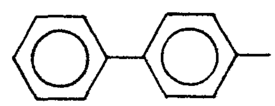 | $CH_3$ | 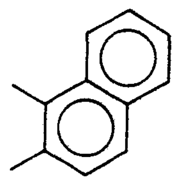 | etwas rotstichig blau |
| 4 | 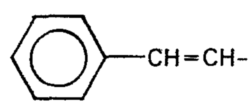 | H | 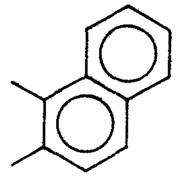 | blau |

**Patentansprüche**

1. Triazolylstyrylverbindugnen der Formel

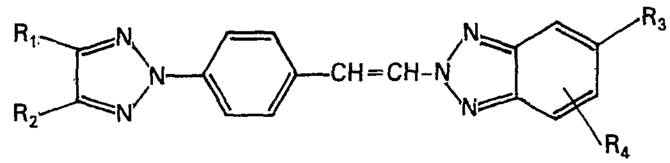

(I)

in welcher $R_1$ für Wasserstoff, $C_1$–$C_4$-Alkyl oder $R_2$,
$R_2$ für Phenyl, Tolyl, Chlorphenyl, Cyclohexyl, Xylyl, Biphenylyl oder Styryl stehen, und
$R_3$ und $R_4$ gemeinsam einen angular anellierten Benzolring bilden.

2. Triazolylstyrylverbindungen gemäss Anspruch 1, dadurch gekennzeichnet, dass $R_1$ für Wasserstoff, $C_1$–$C_2$-Alkyl oder Phenyl
$R_2$ für Phenyl oder Styryl,
$R_3$ und $R_4$ gemeinsam für die restlichen Glieder einen angular ankondensierten Benzolring stehen.

3. Verwendung der Triazolylstyrylverbindungen gemäss der Ansprüche 1–2 zum Weisstönen von organischen Materialien

**Claims**

1. Triazolylstyryl compounds of the formula

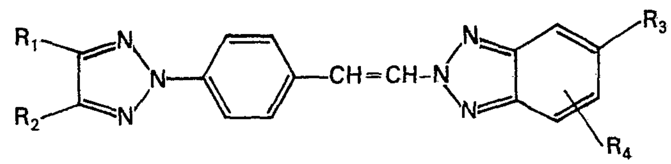

(I)

in which $R_1$ represents hydrogen, $C_1$–$C_4$-alkyl or $R_2$,
$R_2$ represents phenyl, tolyl, chlorophenyl, cyclohexyl, xylyl, biphenylyl or styryl, and
$R_3$ and $R_4$ together form an angularly fused benzene ring.

2. Triazolylstyryl compounds according to claim 1, characterised in that $R_1$ represents hydrogen, $C_1$–$C_2$-alkyl or phenyl
$R_2$ represents phenyl or styryl,
$R_3$ and $R_4$ together represent the remaining members of an angularly fused benzene ring.

3. Use of the triazolylstyryl compounds according to claims 1–2 for whitening organic materials.

**Revendications**

1. Composés triazolylstyryliques de formule

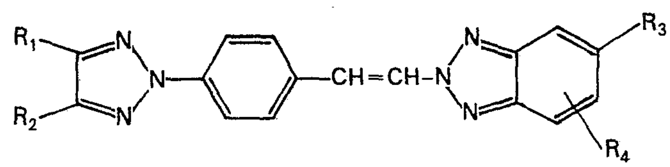

(I)

dans laquelle $R_1$ représente l'hydrogène, un groupe alkyle en $C_1$–$C_4$ ou $R_2$,
$R_2$ représente un groupe phényle, tolyle, chlorophényle, cyclohexyle, xylyle, biphénylyle ou styryle et
$R_3$ et $R_4$ forment ensemble un cycle benzénique condensé en disposition angulaire.

2. Composés triazolylstyryliques selon la revendication 1, caractérisés en ce que $R_1$ représente l'hydrogène, un groupe alkyle en $C_1$–$C_2$ ou phényle,
$R_2$ représente un groupe phényle ou styryle,
$R_3$ et $R_4$ forment ensemble les chaînons restants d'un cycle benzénique condensé en disposition angulaire.

3. Utilisation des composés triazolylstyryliques selon les revendications 1 et 2 pour l'azurage de matières organiques.